(19) Europäisches Patentamt
European Patent Office
Office européen des brevets

(11) Veröffentlichungsnummer: **0 025 870**
**B1**

# (12) EUROPÄISCHE PATENTSCHRIFT

(45) Veröffentlichungstag der Patentschrift: **02.03.83**

(51) Int. Cl.³: **C 07 C 49/587**, C 07 C 45/65, A 61 K 7/46

(21) Anmeldenummer: **80104890.1**

(22) Anmeldetag: **16.08.80**

(54) **Cyclopentadecen-8-on-1, Verfahren zu seiner Herstellung und seine Verwendung als Riechstoff.**

(30) Priorität: **28.08.79 DE 2934678**

(43) Veröffentlichungstag der Anmeldung: **01.04.81 Patentblatt 81/13**

(45) Bekanntmachung des Hinweises auf die Patenterteilung: **02.03.83 Patentblatt 83/9**

(84) Benannte Vertragsstaaten: **CH DE FR GB IT LI NL**

(56) Entgegenhaltungen:
**DE-B-1 668 054**
**US-A-2 790 005**

(73) Patentinhaber: **HAARMANN & REIMER GMBH, Postfach 138, D-3450 Holzminden (DE)**

(72) Erfinder: **Bauer, Kurt, Dr., Corveyblick 41, D-3450 Holzminden (DE)**
Erfinder: **Körber, Alfred, Dr., Bismarckstrasse 4, D-3450 Holzminden (DE)**
Erfinder: **Oelkers, Egon, Rotdornstrasse 15, D-3454 Bevern (DE)**
Erfinder: **Bork, Karl-Heinz, Kurze Breite 10, D-3457 Deensen (DE)**

(74) Vertreter: **Dill, Erwin, Dr. et al, c/o BAYER AG Zentralbereich Patente Marken und Lizenzen Bayerwerk, D-5090 Leverkusen 1 (DE)**

**EP 0 025 870 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt. wenn die Einspruchsgebühr entrichtet worden ist (Art. 99(1) Europäisches Patentübereinkommen).

BUNDESDRUCKEREI BERLIN

Cyclopentadecen-8-on-1, Verfahren zu seiner Herstellung
und seine Verwendung als Riechstoff

Die Erfindung betrifft Cyclopentadecen-8-on-1, ferner ein Verfahren zu seiner Herstellung.

Aus der US 2 790 005 ist ein Verfahren zur Herstellung von Civetone bekannt (Cycloheptadecen-9-on-1). Civetone ist ein Moschusriechstoff der an Zibet erinnert.

Das Herstellungsverfahren ist dadurch gekennzeichnet, daß man eine Verbindung der allgemeinen Formel

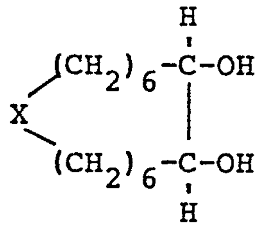

(I)

in der

X für eine Keto- oder Ketalgruppe steht, mit einer Lösung von Bromwasserstoffsäure in Eisessig in die Bromacetoxiverbindung und diese durch Behandeln mit Zinkstaub in Cyclopentadecen-8-on-1 überführt.

Außerdem betrifft die Erfindung die Verwendung von Cyclopentadecen-8-on-1 als Riechstoff. Cyclopentadecen-8-on-1 ist ebenfalls ein Moschusriechstoff. Im Gegensatz zu Civetone hat er jedoch eine deutlich natürlichere Moschusnote und zusätzlich holzige und leicht süßlich-erogene Akzente.

Vertreter der Verbindungen der allgemeinen Formel (I) sind 9-Oxo-Cyclopentadecan-1,2-diol und seine Ketale, z. B. das Dimethyl-, Diethyl-, vorzugsweise aber das Ethylenketal. Die Überführung der Ketogruppe in die Ketalgruppe erfolgt unter den in Tetrahedron 20, 2601 (1964) beschriebenen Bedingungen.

Die Herstellung der Verbindungen der Formel (I) kann nach folgendem Verfahren erfolgen: Korksäurehalbesterchlorid wird in Gegenwart der äquivalenten Menge Triethylamin einer Selbstkondensation unterworfen und das Kondensationsprodukt decarboxyliert. Die so erhaltene 8-Oxo-pentadecandisäure wird in den Ethylenester überführt und dieser mit Ethylenglykol ketalisiert. Die Acyloinkondensation des 8-Alkylendioxypentadecandisäureesters und die nachfolgende Reduktion des Acyloinkondensationsproduktes mit Lithiumaluminiumhydrid liefert die Verbindungen der Formel (I) in der X für eine Ketalgruppe steht. Die Ketalgruppe kann durch saure Hydrolyse in die Ketogruppe überführt werden.

Das erfindungsgemäße Cyclopentadecen-8-on-1 ist ein wertvoller Riechstoff, der in einer Riechstoffkomposition bereits im Angeruch eine kräftige, natürliche Moschusnote entwickelt, die bei langer Haftung ihren Charakter beibehält. Er vereinigt also den starken Angeruch des Cyclopentadecanolids mit der Stabilität des Cyclopentadecanons. Außerdem weist das Cyclopentadecen-8-on-1 eine angenehme, an Sandelholzöl ostindisch erinnernde Note auf, die in Verbindung mit der Moschusnote parfümistisch sehr interessant ist.

Die erfindungsgemäße Verbindung wird in Mischungen mit anderen Riechstoffen in Riechstoffkompositionen, z. B. in Mengen von 0,01 bis 25 Gew.-%, bezogen auf das Gesamtgewicht eingesetzt.

Das Anwendungsgebiet der erfindungsgemäßen Verbindung ist wegen ihres harmonischen Geruchstyps und ihrer vorteilhaften anwendungstechnischen Eigenschaften, z. B. der Haftfestigkeit und der Stabilität gegenüber aggressiven Medien, ungewöhnlich groß. Sie eignet sich für die Verwendung in Parfümkompositionen für die verschiedenen Fertigungsprodukte z. B. für hochwertige Kosmetika, für Feinparfümerieartikel wie Extraits, Seifen, Deo-Spray, Shampoos, Schaumbäder und für Detergentien.

Beispiel 1

59 g (0,196 Mol) 9-Ethylendioxi-cyclopentadecan-1,2-diol werden mit 375 ml einer 30%igen Lösung von Bromwasserstoffsäure in Eisessig 24 Stunden bei Raumtemperatur gerührt und dann 3 Stunden auf 65° C erwärmt. Nach Zugabe von 60 g Essigsäureanhydrid wird die Mischung weitere 3 Stunden auf 65° C gehalten. Nach dem Abkühlen wird überschüssige Bromwasserstoffsäure mit 135 g Natriumacetat in 1 Liter Wasser neutralisiert und die Reaktionsmischung mit Petroläther extrahiert. Nach dem Abdestillieren des Lösungsmittels erhält man 63 g 2-Acetoxi-9-oxo-cyclopentadecan-1-bromid.

Diese 63 g 2-Acetoxy-9-oxo-cyclopentadecan-1-bromid werden ohne weitere Reinigung in 480 ml absolutem Methanol gelöst und nach Zugabe von 45 g Zinkstaub unter Rühren 12 Stunden auf

Rückflußtemperatur erhitzt. Nach dem Abdestillieren des Methanols wird der Rückstand in Petrolether aufgenommen und filtriert. Das Filtrat wird zunächst mit verdünnter Essigsäure, dann mit Wasser säurefrei gewaschen. Der nach Abziehen des Lösungsmittels verbleibende Rückstand wird fraktioniert destilliert. Man erhält 20 g Cyclopentadecen-8-on-1 mit einem Schmelzpunkt von 38—40° C.

Das als Ausgangsmaterial verwendete 9-Ethylendioxi-cyclopentadecan-1,2-diol wird nach folgendem Verfahren hergestellt:

Zu einer Lösung von 860 g (3,9 Mol) Korksäureethylesterchlorid in 8 l Toluol werden unter Rühren und Kühlen 402 g (3,9 Mol) Triethylamin so zugetropft, daß die Innentemperatur 40° C nicht übersteigt. Nach beendeter Zugabe wird die Reaktionsmischung 48 Stunden bei Raumtemperatur gerührt. Dann wird das ausgefallene Triethylammoniumchlorid abgetrennt, das Filtrat eingeengt und mit Wasser gewaschen. Zu der mit 360 ml Ethanol und 40 ml Wasser verdünnten organischen Phase wird unter Rühren und Kühlen eine Lösung von 720 g Kaliumhydroxid in 3200 ml Ethanol und 400 ml Wasser zugetropft. Dann wird 6 Stunden auf Rückflußtemperatur erhitzt. Anschließend wird bei schwachem Vakuum das Ethanol weitgehend abdestilliert, der Rückstand mit Wasser verdünnt und mit 15%iger Salzsäure angesäuert. Das ausgefallene Reaktionsprodukt wird abfiltriert. Man erhält 546 g 8-Oxo-pentadecandisäure (Fp.: 115° C).

Diese 546 g 8-Oxo-pentadecandisäure werden ohne weitere Reinigung in 3036 ml Ethanol gelöst und mit 86,2 g konzentrierter Schwefelsäure versetzt. Das Reaktionsgemisch wird 6 Stunden auf Rückflußtemperatur erhitzt. Nach dem Aufarbeiten werden durch fraktionierte Destillation 601 g 8-Oxo-pentadecandisäurediethylester (Fp.: 32° C) erhalten.

171 g (0,5 Mol) dieses Diesters werden mit 230 ml Ethylenglykol und 1 g p-Toluolsulfonsäure in 6,3 l Benzol gelöst und solange auf Rückflußtemperatur erhitzt, bis sich kein Wasser mehr abscheidet. Dann wird die benzolische Lösung neutralgewaschen und von überschüssigem Ethylenglykol befreit. Nach dem Abziehen des Lösungsmittels verbleiben 183 g 8-Ethylendioxi-pentadecandisäurediethylester (Kp.: 190—195° C/0,66 mbar).

Die Lösung von 100 g (0,26 Mol) dieses Ketalesters in 100 ml Xylol wird innerhalb von 5 Stunden bei Rückflußtemperatur zu einer Suspension von 24 g Natrium in 1688 ml Xylol getropft. Nach dem Abkühlen werden 54 g Eisessig in 55 ml Xylol zugetropft. Das Reaktionsgemisch wird mit Wasser neutralgewaschen und das Lösungsmittel abgezogen. Die Destillation des Rückstandes ergibt 60,4 g 9-Ethylendioxid-2-hydroxi-cyclopentadecanon-1 mit einem Siedepunkt von 183—186° C/0,66 mbar.

Die Lösung von 60 g (0,2 Mol) des 9-Ethylendioxi-2-hydroxi-cyclopentadecanon-1 in 500 ml Ether wird innerhalb von 30 Minuten zu einer Suspension von 21 g Lithiumaluminiumhydrid in 1000 ml Ether unter Eiskühlung getropft. Die Reaktionsmischung wird 10 Stunden auf Rückflußtemperatur erhitzt. Anschließend wird überschüssiges Lithiumaluminiumhydrid mit feuchtem Ether zersetzt und das Reaktionsgemisch mit Wasser neutralgewaschen. Nach Abziehen des Lösungsmittels erhält man 59 g 9-Ethylendioxi-cyclopentadecan-1,2-diol.

## Beispiel 2

Eine frische Unisex-Typ-Komposition wird durch Mischen der folgenden Komponenten hergestellt:

| | |
|---|---|
| 100 | Linalylacetat |
| 50 | Citronenöl italienisch |
| 10 | Mandarinenöl |
| 10 | Ocimenylacetat |
| 20 | Dihydromyrcenol |
| 10 | Lavendelöl Barreme |
| 5 | Styrolylacetat |
| 5 | Galbanumöl |
| 2 | Acetaldehyd-di-cis-3-hexenyl-acetal |
| 30 | α-Hexylzimtaldehyd |
| 10 | Hydroxicitronellal |
| 5 | Benzylacetat |
| 10 | Hexylsalicylat |
| 5 | Isoamylsalicylat |
| 5 | Geraniumöl Bourbon |
| 1 | Dimethylanthranilat |
| 60 | Cedrylketon |
| 40 | Cedrylacetat |
| 5 | Evernyl (Handelsprodukt der Fa. Roure-Betrand) |
| 3 | Fixateur Ambra |
| 10 | Cumarin |
| 5 | Acetylvanillin 10%ig in Diethylphthalat |
| 2 | Heliotropin |
| 403 | Gewichtsteile |

Durch Zugabe von 100 Gewichtsteilen Cyclopentadecen-8-on-1 erhält die Komposition bereits im Angeruch eine besondere Strahlkraft und neben einer warmen Moschusnote einen angenehm weichen, holzigen Charakter.

**Patentansprüche**

1. Cyclopentadecen-8-on-1.
2. Verfahren zur Herstellung Cyclopentadecen-8-on-1, dadurch gekennzeichnet, daß man eine Verbindung der allgemeinen Formel

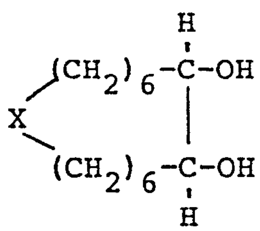

in der

X für eine Keto- oder Ketalgruppe steht, mit einer Lösung von Bromwasserstoffsäure in Eisessig in die Bromacetoxiverbindung und diese durch Behandeln mit Zinkstaub in Cyclopentadecen-8-on-1 überführt.

3. Verwendung von Cyclopentadecen-8-on-1 als Riechstoff.

**Claims**

1. Cyclopentadec-8-en-1-one.
2. Process for the preparation of cyclopentadec-8-en-1-one, characterised in that a compound of the general formula

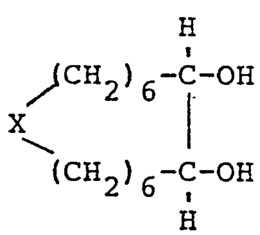

in which

X represents a keto group or ketal group, is converted into the bromoacetoxy compound with a solution of hydrobromic acid in glacial acetic acid, and this compound is converted into cyclopentadec-8-en-1-one by treatment with zinc dust.

3. Use of cyclopentadec-8-en-1-one as a fragrance.

**Revendications**

1. La cyclopentadécène-8-one-1.

2. Procédé pour la production de cyclopentadécène-8-one-1, caractérisé en ce que l'on transforme un composé de formule générale

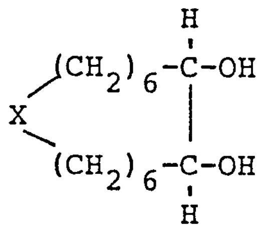

dans laquelle

X représente un groupe céto ou un groupe acétal, en composé bromoacétoxy avec une solution d'acide bromhydrique dans l'acide acétique et on transforme ce composé en cyclopentadécène-8-one-1 par traitement avec la poudre de zinc.

3. Utilisation de la cyclopentadécène-8-one-1 comme parfum.